# EUROPEAN PATENT SPECIFICATION

(11) **EP 2 083 748 B1**
(45) Date of publication and mention of the grant of the patent: **18.04.2012**
(21) Application number: 07824298.9
(22) Date of filing: 23.10.2007
(51) Int. Cl.: A61F 2/06

(54) **HELICAL STENT GRAFT**
SPIRALFÖRMIGES STENT-GRAFT
GREFFE DE STENT HÉLICOÏDAL

(30) Priority: 23.10.2006 GB 0621048
(43) Date of publication of application: 05.08.2009
(73) Proprietor: Anson Medical Limited, Didcot Oxfordshire, OX11 7HJ (GB)
(72) Inventor: KEEBLE, Duncan, Oxfordshire OX11 7HJ (GB); GODDARD, Robert William, Oxfordshire OX11 7HJ (GB)
(74) Representative: Tollett, Ian
(86) International application number: PCT/GB2007/004051
(87) International publication number: WO 2008/050115

(56) References cited:
- EP-A- 1 666 003
- EP-A- 1 700 581
- WO-A-01/82836
- US-A- 5 665 115
- US-A1- 2002 040 238
- US-A1- 2003 212 450
- US-A1- 2004 034 403
- US-B1- 6 309 413

## Description

The present invention relates to a stent graft and in particular to a helical stent graft for use in the thoracic artery.

The use of stent-grafts in minimally invasive surgery is well known in the treatment of aortic aneurysms and aneurysms of other blood vessels. Stent grafts, sometimes also known as covered stents, are also used in the gastro-intestinal tract and other tubular structures of the body where the vessel-wall has become diseased or defective in some other way.

Stent grafts are tubular structures usually constructed from two basic components: a reinforcement structure the purpose of which is to lock the stent-graft to the vessel's walls and to keep open the lumen of the vessel; and a tubular graft, which is at least partially attached to the reinforcement structure and the purpose of which is to contain the substance which would normally flow through the natural vessel.

Many designs of reinforcement structure exist; some are complex and expensive to manufacture, such as laser cut tubing, while others formed from simple wire are cheaper to manufacture.

Document US2003/0212450 discloses a stent comprising multiple helical stent sections connected via elastic tubular sections. The size and properties of each stent section may be varied independently.

Document US6309413 discloses a stent graft, wherein a tubular graft is reinforced by a plurality of stent elements spaced along the graft. The adjoining graft regions are supported primarily via their proximity to the regions directly overlying the stents.

One particular design, in which the reinforcement structure is a long helix of wire, is both economical to manufacture and has the particular benefit of being outstandingly flexible. However, there are a number of problems associated with helically reinforced stent grafts:
A first problem is that the device can be weakened significantly by a single fracture of the wire which can allow the reinforcement wire to unravel from the device.
Unravelling can also be caused by slack in or damage to the stitches enabling the wire to move in the stitches and away from the sealing zones (even if the wire does not break). Either event can result in loss of radial force at the sealing zones (i.e. the ends of the stent graft) and potentially a leak.
A second problem is the limitation of using the same wire throughout the device. This prevents the selection of wires optimised to different zones of the implant.
A third is the tendency of the reinforcement loops of the helix to fall over separately, in a similar manner to a line of dominoes, resulting in the collapse of the tubular stent graft.

The present invention is intended to at least partially address the problems of helical stent grafts and is not relevant therefore to non-helical stent grafts.

In a first aspect of the present invention there is provided a stent graft comprising graft material in the form of a tube and reinforcing material for the graft material, the reinforcing material being in at least two separate sections, each section comprising a continuous integral piece of material, wherein each section of reinforcing material is in the form of a helix with a longitudinal axis substantially parallel to the longitudinal axis of the tube.

In a preferred embodiment, at least two wires, with appropriate termination, are used in sequence along the length of the stent graft. Such a construction limits the extent to which the reinforcement wire can unravel in the event of a wire break or attachment failure - for example failure of one stent section does not result in failure of the other stent section. It also permits use of wires of different properties within the same stent graft. Fixation loops may be provided at the ends of the wires to provide increased stability to the structure, reducing the risk of 'domino' collapse.

In a practical embodiment of a stent-graft, at least two reinforcement elements are stitched to the surface of the graft material and it is preferable that the end of one helix is attached to the graft material as close as possible to or abutting the beginning of a next helix in order to avoid a discontinuity in the reinforcement of the graft.

Alternatively, a separation can be introduced between the end of one helix and the beginning of the next so as to create a zone along the length of the stent graft with reduced stiffness. This separation may be from a sixth to a half of the diameter of the reinforcing material.

In a particularly preferred embodiment the stent graft of the present invention has three sections of reinforcing material, namely a middle section and two end sections. Preferably, the length of each end section measured in a straight line along the longitudinal axis of the stent graft is independently from 5% to 33% of the total length of the stent graft. The end sections may however be of substantially the same length.

In one embodiment, the length of each end section measured in a straight line along the longitudinal axis of the stent graft is about 20% of the total length of the stent graft.

By way of illustration, consider aortic stent grafts which have diameters which lie in the range 20mm to 45mm. In a preferred embodiment of the stent graft, the pitch of the helix is in the range one sixth to one half the diameter of the stent graft; in the range of practical aortic stent grafts, this would equal a pitch in the range approximately 3mm to 23mm. In some circumstances, it is desirable to reduce the pitch and practical devices can be made from helices with a pitch equal to the diameter of the wire itself for at least part of the overall length of the stent graft.

The ends of stent grafts are particularly critical because they provide the seal between the stent graft and the native vessel. It is preferable that at least the reinforcement components of the end seals are separate from the body of the graft so that damage to the body will not affect the critical seal zones. The minimum number of separate helices in this construction is three.

A large number of individual helices can be attached to the surface of a stent graft; in designs employing a single turn helix with a pitch of 3mm, a graft of 200mm overall length will employ approximately 67 separate helices.

As noted above, the seal zones at either end of a stent graft are critical areas and with the design here disclosed, wires of different stiffnesses, finishes, diameters or even materials can be used just in these critical areas. Moreover, there is merit in placing reinforcement structures within the lument of stent grafts at the seal zones to allow direct contact between the graft fabric and the native vessel. In other parts of the stent graft, it is preferable that the reinforcement wires lie on the outside of the graft so as to minimise the structures within the graft that can disrupt, for examples, the introduction of guide wires or the flow of blood.

In an alternative aspect not covered by the claims there is provided a stent graft comprising graft material and reinforcing material for the graft material, wherein the reinforcing material is formed from a single continuous integral piece of material having at least one integral loop formed along its length for attaching the reinforcing material to the graft material.

Preferably, the reinforcing material has a plurality of integral loops along it length. It may be formed of a continuous wire formed into a helix as described above.

A usual method of attaching reinforcement wires to the surface of stent grafts is to use sewn stitches, formed either by hand or by machine. Machine stitching is usually either chain stitch or lock stitch, depending upon whether or not a second yam is used. Both stitches are prone to 'running' if a yam is broken, chain stitch being significantly more prone to this failure mode than lock stitch. Where machine stitches are used in the construction of helical and other wire stent grafts, it is preferable to stitch backwards at frequent intervals over the construction of the stent graft. Back stitching (ie stitching) over previously formed stitches) is an effective way of locking stitches in place even in the event of yam breakage.

There is further disclosed a method for implantation in the thoracic artery of a stent graft as defined above.

A preferred embodiment of the invention will now be described with reference to the drawing, in which:

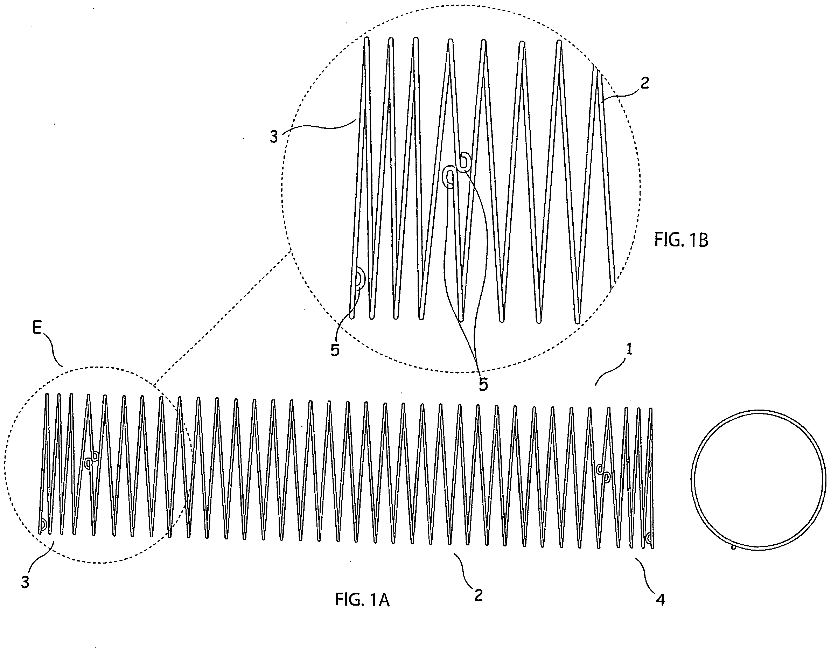

Figure 1A depicts a thoracic stent according to the invention (with the graft material omitted for clarity);
Figure 1B is an enlarged view of the end section E of the stent of Figure 1A.

Figures 1A and 1B illustrate the fundamental construction of the reinforcement wires which form the reinforcement elements of the stent graft. Thoracic stent 1 is formed of three sections - mid-section stent 2, proximal stent 3 and distal stent 4 which are independent of each other. The fundamental shape of all three sections is a wire-form helix, usually containing at least one and usually several full turns of wire with a pitch of at least the diameter of the wire. The two free ends of each helix carry a formation or additional component intended to facilitate attachment of the free ends of the wire to the graft material so as to avoid movement of the wire or damage by the wire to the graft material. A simple formation involves constructing loop 5 at each of the ends of the wire, although alternative solutions, such as forming a series of 'S' bends or attaching a preformed fixable shape, are also effective. The loops 5 are pre-fractured to lessen the risk of unexpected fracturing occurring in use and are attached to the graft material (not shown).

In use, proximal stent 3 and distal stent 4 provide a seal with the aorta and mid-section stent 2 provides support for the graft within the aneurysm. If mid-section stent 2 fails and begins to unravel then this failure is restricted due to the separation between mid-section stent 2 and proximal stent 3 and distal stent 4.

## Claims

1. A stent graft (1) comprising graft material in the form of a tube and reinforcing material for the graft material, the reinforcing material being in at least two separate sections, each section (2, 3, 4) comprising a continuous integral piece of material, wherein each section of reinforcing material is in the form of a helix with a longitudinal axis substantially parallel to the longitudinal axis of the tube,
**characterised in that** the first section abuts or is as close as possible to the second section in order to avoid a discontinuity in the reinforcement of the graft material.

2. A stent graft as claimed in claim 1 wherein the two helices are in the form of a single helix with a section removed to create a gap between the first and second helix so as to create a zone of reduced stiffness along the length of the stent graft.

3. A stent graft as claimed in claim 2, wherein the gap is from a sixth to a half of the diameter of the reinforcing material.

4. A stent graft as claimed in any preceding claim wherein each section of reinforcing material has a fixation loop (5) at each end.

5. A stent graft as claimed in any preceding claim which has three sections of reinforcing material, a middle section (2) and two end sections (3, 4).

6. A stent graft as claimed in claim 5, wherein the length of each end section measured in a straight line along the longitudinal axis of the stent graft is independently from 5% to 33% of the total length of the stent graft.

7. A stent graft as claimed in claim 5 or 6, wherein the end sections are of substantially the same length measured in a straight line along the longitudinal axis of the stent graft.

8. A stent graft as claimed in claim 7, wherein the length of each end section measured in a straight line along the longitudinal axis of the stent graft is about 20% of the total length of the stent graft.

9. A stent graft as claimed in any of claims 1 to 5 which has only two sections of reinforcing material, the sections being of substantially equal length measured in a straight line along the longitudinal axis of the stent graft.

## Patentansprüche

1. Stent-Graft (1) mit einem Graft- oder Transplantatmaterial in der Form eines Rohrs und einem verstärkenden Material für das Transplantatmaterial, wobei das verstärkende Material in wenigstens zwei separaten Abschnitten vorliegt, wobei jeder Abschnitt (2, 3, 4) ein durchgängiges, einstückiges Stück an Material aufweist, wobei jeder Abschnitt des verstärkenden Materials in der Form einer Wendel mit einer Längsachse im Wesentlichen parallel zur Längsachse des Rohres ist,
**dadurch gekennzeichnet, dass** der erste Abschnitt den zweiten Abschnitt abstützt oder so nahe wie möglich am zweiten Abschnitt ist, um eine Diskontinuität in der Verstärkung des Transplantatmaterials zu vermeiden.

2. Stent-Graft nach Anspruch 1, wobei die beiden Wendeln in der Form einer einzelnen Wendel mit einem entfernten Abschnitt sind, um eine Lücke zwischen der ersten und der zweiten Wendel zu schaffen, um eine Zone verringerter Steifigkeit entlang der Länge des Stent-Graft zu schaffen.

3. Stent-Graft nach Anspruch 2, wobei die Lücke zwischen einem Sechstel bis zur Hälfte des Durchmessers des verstärkenden Materials beträgt.

4. Stent-Graft nach einem der vorhergehenden Ansprüche, wobei jeder Abschnitt des verstärkenden Materials eine Fixierungsschlinge oder -schleife (5) an jedem Ende hat.

5. Stent-Graft nach einem der vorhergehenden Ansprüche, wobei er drei Abschnitte an verstärkendem Material aufweist, einen mittleren Abschnitt (2) und zwei Endabschnitte (3, 4).

6. Stent-Graft nach Anspruch 5, wobei die Länge jedes Endabschnitts gemessen in einer geraden Linie entlang der Längsachse des Stent-Grafts unabhängig zwischen 5% bis 33% der Gesamtlänge des Stent-Grafts beträgt.

7. Stent-Graft nach Anspruch 5 oder 6, wobei die Endabschnitte im Wesentlichen die gleiche Länge gemessen in einer geraden Linie entlang der Längsachse des Stent-Grafts haben.

8. Stent-Graft nach Anspruch 7, wobei die Länge jedes Endabschnitts gemessen in einer geraden Linie entlang der Längsachse des Stent-Grafts ungefähr 20% der Gesamtlänge des Stent-Grafts beträgt.

9. Stent-Graft nach einem der Ansprüche 1 bis 5, wobei er nur zwei Abschnitte aus verstärkendem Material hat, wobei die Abschnitte im Wesentlichen gleich lang sind, gemessen in einer geraden Linie entlang der Längsachse des Stent-Grafts.

## Revendications

1. Une endoprothèse (1) comprenant un matériau de greffe en forme de tube et un matériau de renforcement, le matériau de renforcement étant composé d'au moins deux sections séparées, chaque section (2, 3, 4) comprenant une pièce continue faisant partie intégrante du matériau, dans laquelle chaque section du matériau de renforcement a une forme hélicoïdal avec un axe longitudinal substantiellement parallèle à l'axe longitudinal du tube, **caractérisé en ce que** la première section juxtapose ou est le plus près possible de la seconde section de façon à éviter une discontinuité dans le renforcement du matériau de greffe.

2. Une endoprothèse selon la revendication 1, dans laquelle les deux hélices sont en forme d'une seule hélice avec une section supprimée pour créer un espace entre la première et la seconde hélice, de façon à créer une zone de rigidité réduite sur la longueur de l'endoprothèse.

3. Une endoprothèse selon la revendication 2, dans laquelle l'espace est d'un sixième à la moitié du diamètre du matériau de renforcement.

4. Une endoprothèse selon l'une quelconque des précédentes revendications, dans laquelle chaque section du matériau de renforcement a une boucle de fixation (5) à chaque extrémité.

5. Une endoprothèse selon l'une quelconque des précédentes revendications, qui a trois sections de matériau de renforcement, une section centrale (2) et deux sections d'extrémité (3,4).

6. Une endoprothèse selon la revendication 5, dans laquelle la longueur de chaque section d'extrémité mesurée en ligne droite le long de l'axe longitudinal de l'endoprothèse est indépendamment entre 5% et 33% de la longueur total de l'endoprothèse.

7. Une endoprothèse selon l'une des revendications 5 ou 6, dans laquelle les sections d'extrémité sont substantiellement de la même longueur mesurée en ligne droite le long de l'axe longitudinal de l'endoprothèse.

8. Une endoprothèse selon la revendication 7, dans laquelle la longueur de chaque section d'extrémité mesurée en ligne droite le long de l'axe longitudinal de l'endoprothèse est environ 20% de la longueur totale de l'endoprothèse.

9. Une endoprothèse selon l'une des revendications 1 à 5, qui a seulement deux sections de matériau de renforcement, les sections étant substantiellement de longueur équivalente, longueur mesurée en ligne droite le long de l'axe longitudinal de l'endoprothèse.
